# EUROPÄISCHE PATENTSCHRIFT

(11) **EP 1 496 870 B1**
(45) Veröffentlichungstag und Bekanntmachung des Hinweises auf die Patenterteilung: **02.09.2009**
(21) Anmeldenummer: 04716230.0
(22) Anmeldetag: 02.03.2004
(51) Int. Cl.: A61K 9/32

(54) **ARZNEIFORM UND VERFAHREN ZU IHRER HERSTELLUNG**
DOSAGE FORM AND METHOD FOR PRODUCING THE SAME
FORME GALENIQUE ET SON PROCEDE DE PRODUCTION

(30) Priorität: 29.04.2003 DE 10319458
(43) Veröffentlichungstag der Anmeldung: 19.01.2005
(73) Patentinhaber: Evonik Röhm GmbH, 64293 Darmstadt (DE)
(72) Erfinder: PETEREIT, Hans-Ulrich, 64291 Darmstadt (DE); MEIER, Christian Dr., 64295 Darmstadt (DE); SCHULTES, Klaus Dr., 65197 Wiesbaden (DE)
(86) Internationale Anmeldenummer: PCT/EP2004/002061
(87) Internationale Veröffentlichungsnummer: WO 2004/096185

(56) Entgegenhaltungen:
- EP-A- 0 704 207
- WO-A-03/032958
- WO-A-03/072087
- DE-A- 10 013 030

## Beschreibung

Die Erfindung betrifft ein Verfahren zur Herstellung von Arzneiformen, die Arzneiformen selbst, das darin enthaltene Copolymer sowie dessen Verwendung zur Herstellung der Arzneiform.

### Stand der Technik

EP 0 704 208 A2 beschreibt Überzugs- und Bindemittel für darmsaftlösliche Arzneiumhüllungen. Es handelt sich dabei um Copolymerisate aus 10 bis 25 Gew.-% Methacrylsäure, 40 bis 70 Gew.-% Methylacrylat und 20 bis 40 Gew-% Methylmethacrylat. Die Beschreibung erwähnt neben einschichtigen Überzügen auch mehrlagige Überzugssysteme. Diese können aus einem Kern, der z. B. einen basischen oder einen wasserempfindlichen Wirkstoff enthält, bestehen, weisen eine Isolierschicht aus einem anderen Überzugsmaterial, wie Celluloseether, Celluloseester oder einem kationischen Polymethacrylat z. B. von Typ EUDRAGIT®, u. a. auch EUDRAGIT® RS und RL, auf und werden dann zusätzlich mit der oben genannten darmsaftlöslichen Umhüllung versehen.

EP 0 704 207 A2 beschreibt thermoplastische Kunststoffe für darmsaftlösliche Arzneiumhüllungen. Es handelt sich dabei um Mischpolymerisate aus 16 bis 40 Gew.-% Acryl- oder Methacrylsäure, 30 bis 80 Gew.-% Methylacrylat und 0 bis 40 Gew.-% anderen Alkylestern der Acrylsäure und/oder Methacrylsäure. Die Mindestfilmbildungstemperatur (MFT nach DIN 53 778) liegt im Bereich zwischen 0 und 25 °C, so daß die Verarbeitung bei Raumtemperatur ohne Weichmacherzusatz möglich ist. Die Reißdehnung der Filme, gemessen nach DIN 53 455, liegt bei einem Gehalt von höchstens 10 Gew.-% Triethylcitrat in der Regel bei 50 % oder mehr.

### Aufgabe und Lösung

EP 0 704 207 A2 und EP 0 704 208 A2 beschreiben Copolymere zur Herstellung von Arzneiformen auf Basis von (Meth)acrylat-Monomeren, die vergleichsweise flexible Filme bilden und im Bereich höher pH-Werte ab ca, 6,0 für viele Arzneistoffe geeignete Freisetzungsprofile aufweisen.

Die permanent voranschreitende Entwicklung von Arzneiformen verlangt zunehmend nach immer besseren Überzugs- und Bindemitteln. Es werden Überzugs- und Bindemittel entwickelt, die teilweise in Verbindung mit weiteren Zusatzstoffen immer speziellere Freisetzungscharakteristiken, maßgeschneidert auf den jeweiligen Wirkstoff ermöglichen.

Die Entwicklung geht aber andererseits auch dahin, generell den Anteil an Zusatzstoffen gegenüber dem eigentlichen Wirkstoff so weit wie möglich zu reduzieren. Bei funktionell überzogenen Arzneiformen ist man deshalb bestrebt, die Dicke der Überzugsschicht zu reduzieren. Dies hat seine Grenzen zum einen in der angestrebten Freisetzungscharakteristik für den Arzneistoff selbst und zum anderen in der mechanischen Belastbarkeit der Überzüge. Bei sehr dünnen Überzügen besteht stets die Gefahr der mechanischen Beschädigung bei der Herstellung oder der Lagerung.

Weiterhin werden heute bereits viele Wirkstoffe in Form von multipartikulären Arzneiformen bereitgestellt. Durch die Menge der in einer Arzneiform enthaltenen Partikel, gelingt es Schwankungen in der Dosierung, bedingt durch schwankende Überzugsdicken der funktionellen Überzugsschichten, einzuengen. Da die Herstellung multipartikulärer Arzneiformen durch Verpressen von überzogenen wirkstoffhaltigen Pellets zusammen mit Hilfsstoffen unter teilweise hohen Drücken erfolgt, kommen viele ansonsten sehr geeigneter polymerer Überzugsmittel für diese Arzneiform nicht in Frage, da sie den mechanischen Belastungen nicht, nicht ausreichend sicher oder nur dann standhalten, wenn die Überzuge übermäßig dick aufgetragen werden.

EP 0 704 207 A2 und EP 0 704 208 A2 beschreiben Copolymere zur Herstellung von Arzneiformen auf Basis von (Meth)acrylat-Monomeren, die vergleichsweise flexible Filme bilden und im Bereich höher pH-Werte ab ca, 6,0 für viele Arzneistoffe geeignete Freisetzungsprofile aufweisen. Wie sich zeigte sind die EP 0 704 207 A2 konkret beschriebenen Copolymere, zumindest gegenüber höheren mechanischen Belastungen nur wenig widerstandfähig. Die in EP 0 704 208 A2 beschriebenen Polymere lösen sich jedoch erst oberhalb pH 6,5, d.h. in tieferen Abschnitten des Darms. Sie sind daher nicht für Wirkstoffe geeignet, deren Resorption bevorzugt in den oberen Darmbereichen erfolgt.

Es wurde als Aufgabe gesehen, darmsaftlösliche Arzneiformen bereitzustellen, die den Wirkstoff erst ab etwa pH 5;8 bis ca. 6,0 freisetzen und die ohne übermäßigen Weichmacherzusatz gleichzeitig mechanisch stabile, nicht klebrige Filme ausbilden. Insbesondere sollten Zubereitungen bereitgestellt werden, die den hohen mechanischen Anforderungen bei der Herstellung multipartikulärer Arzneiformen genügen.

Die Aufgabe wird gelöst durch ein Verfahren zur Herstellung einer überzogenen Arzneiform oder einer Arzneiform in Form einer wirkstoffhaltigen Matrix, indem man ein Copolymer, einen pharmazeutischen Wirkstoff, einen gegebenenfalls vorhandenen Kern und/oder pharmazeutisch übliche Zuschlagstoffe in an sich bekannter Weise durch Schmelzen, Spritzguß, Extrusion, Feuchtgranulieren, Gießen, Tauchen, Ausstreichen, Aufsprühen oder Verpressen zu einer überzogenen Arzneiform und/oder zu einer wirkstoffhaltigen Matrix verarbeitet, dadurch gekennzeichnet, daß man ein Copolymer einsetzt, welches sich aus
20 bis 33 Gew.-% Methacrylsäure und/oder Acrylsäure,
5 bis 30 Gew.-% Methylacrylat und
20 bis 40 Gew.-% Ethylacrylat und
größer 10 bis 30 Gew.-% Butylmethacrylat und
gegebenenfalls
0 bis 10 Gew.-% weiteren vinylisch copolymerisierbarer Monomeren,
wobei sich die Anteile zu 100 Gew.-% addieren,
zusammensetzt, mit der Maßgabe, daß die Glastemperatur des Copolymers nach ISO 11357-2, Punkt 3.3.3, 55 bis 70 °C beträgt.

Die Erfindung betrifft weiterhin die Arzneiform selbst sowie das Copolymer und dessen Verwendung zu Herstellung der Arzneiform.

### Ausführung der Erfindung

Die Erfindung betrifft ein Verfahren zur Herstellung einer Arzneiform als Tablette, Pellets enthaltende Arzneiform und/oder in Form einer wirkstoffhaltigen Matrix, wobei die Tabletten, Pellets, und/oder die wirkstoffhaltige Matrix einen pharmazeutischen Wirkstoff und ein Copolymer als Überzugs- und/oder Bindemittel, sowie gegebenenfalls einen Kern und pharmazeutisch übliche Zuschlagstoffe enthalten, indem man das Copolymer, den pharmazeutischen Wirkstoffs, den gegebenenfalls vorhandenen Kern und/oder die pharmazeutisch üblichen Zuschlagstoffe in an sich bekannter Weise durch Schmelzen, Spritzguß, Extrusion, Feuchtgranulieren, Gießen, Ausstreichen, Aufsprühen oder Verpressen zu Tabletten oder Pellets und/oder einer wirkstoffhaltigen Matrix verarbeitet.

### Copolymer

Wesentlich für die Erfindung ist die Verwendung eines Copolymers, welches sich aus
20 bis 33 Gew.-% Methacrylsäure und/oder Acrylsäure,
5 bis 30 Gew.-% Methylacrylat und
20 bis 40 Gew.-% Ethylacrylat und
größer 10 bis 30 Gew.-% Butylmethacrylat und gegebenenfalls
0 bis 10 Gew.-% weiteren vinylisch copolymerisierbarer Monomeren,
wobei sich die Anteile der Monomeren zu 100 Gew.-% addieren,
zusammensetzt, mit der Maßgabe, daß die Glastemperatur des Copolymers (glass transition temperature) nach ISO 11357-2, Punkt 3.3.3 (midpoint temperature *T*_{mg}), 55 bis 70 °C beträgt.

Das Copolymer setzt sich insbesondere zusammen aus radikalisch polymerisierten Einheiten von
20 bis 33, bevorzugt 25 bis 32, besonders bevorzugt 28 bis 31 Gew.-% Methacrylsäure oder Acrylsäure, bevorzugt ist Methacrylsäure,
5 bis 30, bevorzugt 10 bis 28, besonders bevorzugt 15 bis 55 Gew.-% Methylacrylat,
20 bis 40, bevorzugt 25 bis 35, besonders bevorzugt 18 bis 22 Gew.-% Ethylacrylat, sowie
größer 10 bis 30, bevorzugt 15 bis 25, besonders bevorzugt 18 bis 22 Gew.-% Butylmethacrylat
zusammen, wobei die Monomerzusammensetzung so gewählt wird, daß die Glastemperatur des Copolymers 55 bis 70 °C, bevorzugt 59 bis 66, besonders bevorzugt 60 bis 65 °C beträgt.

Unter Glastemperatur wird hier insbesondere die midpoint temperature *T*_{mg} nach ISO 11357-2, Punkt 3.3.3, verstanden. Die Messung erfolgt ohne Weichmacherzusatz, bei Restmonomergehalten (REMO) von weniger als 100 ppm, bei einer Aufheizrate von 10 °C/min und unter Stickstoffatmosphäre.

Das Copolymer besteht bevorzugt in wesentlichen bis ausschließlich, zu 90, 95 oder 99 bis 100 Gew.-%, aus den Monomeren Methacrylsäure, Methylacrylat, Ethylacrylat und Butylmethacrylat in den oben angegebenen Mengenbereichen.

Es können jedoch zusätzlich, ohne daß dies zu einer Beeinträchtigung der wesentlichen Eigenschaften führen muß, geringe Mengen im Bereich von 0 bis 10, z. B. 1 bis 5 Gew.-% weiterer vinylisch copolymerisierbarer Monomere, wie z. B. Methylmethacrylat, Butylacrylat, Hydroxyethylmethacrylat, Vinylpyrrolidon, Vinylmalonsäure, Styrol, Vinylalkohol, Vinylacetat und/oder deren Derivate enthalten sein.

### Herstellung des Copolymers

Die Herstellung des Copolymers kann in an sich bekannter Weise durch radikalische Polymerisation der Monomeren erfolgen (siehe z. B. EP 0 704 207 A2 und EP 0 704 208 A2). Das erfindungsgemäße Copolymer ist in an sich bekannter Weise durch radikalische Emulsionspolymerisation in wäßriger Phase in Gegenwart von vorzugsweise anionischen Emulgatoren herstellbar, beispielsweise nach dem in DE-C 2 135 073 beschriebenen Verfahren.

Das Copolymerisat kann nach gängigen Verfahren der radikalischen Polymerisation kontinuierlich oder diskontinuierlich (Batch-Verfahren) in Gegenwart radikalbildender Initiatoren und gegebenenfalls Reglern zur Einstellung des Molekulargewicht in Substanz, in Lösung, durch Perlpolymerisation oder in Emulsion hergestellt werden. Das mittlere Molekulargewicht Mw (Gewichtsmittel, bestimmt z. B. durch Messung der Lösungsviskosität) kann z. B. im Bereich von 80.000 bis 1.000.000 (g/mol) liegen. Bevorzugt ist die Emulsionspolymerisation in wäßriger Phase in Gegenwart wasserlöslicher Initiatoren und (vorzugsweise anionischer) Emulgatoren.
Im Falle der Substanzpolymerisation kann das Copolymer in fester Form durch Brechen, Extrusion, Granulieren oder Heißabschlag erhalten werden.

### Organische Lösung

Das Copolymer kann in Form einer organischen Lösung, z. B. in einer Konzentration von 10 bis 30 Gew.-%, bereitgestellt werden. Als Lösungsmittel können z. B. Aceton, Isopropanol oder Ethanol oder Mischung daraus verwendet werden, die gegebenenfalls Wasseranteile bis etwa 10 Gew.-% enthalten können. Bevorzugt sind jedoch wäßrige Dispersionen.

### Dispersionen

Das Emulsionspolymerisat wird vorzugsweise in Form einer 10- bis 50-gew.-prozentigen, insbesondere 20 bis 40-prozentigen wäßrigen Dispersion erzeugt und angewendet. Als Handelsform ist ein Feststoffgehalt von 30 Gew.-% bevorzugt. Für die Verarbeitung ist eine teilweise Neutralisation der Methacrylsäure-Einheiten entbehrlich; sie ist jedoch, beispielsweise in einem Umfang bis zu 5 oder 10 Mol-% möglich, wenn eine Stabilisierung oder Verdickung der Überzugsmitteldispersion erwünscht sein sollte. Der Gewichtsmittelwert Latex-Teilchengröße (Radius) beträgt in der Regel 40 bis 100 nm, vorzugsweise 50 bis 70 nm, was eine verarbeitungstechnisch günstige Viskosität unter 1000 mPa·s gewährleistet. Die Teilchengröße kann durch Laserbeugung, z. B. mit dem Mastersizer 2000 (Fa. Malvern), bestimmt werden.

Bei höheren Neutralisationsgrades z. B. 10 bis 50 Mol.-% oder vollständiger Neutralisation ist es möglich, das Copolymer in einen gelösten Zustand zu überführen

Um eine Lösung des anionischen Copolymers herzustellen ist in der Regel eine teilweise oder vollständige Neutralisation der Säuregruppen nötig. Das anionische Copolymer kann z. B. nach und nach in einer Endkonzentration von 1 bis 40 Gew.-% in Wasser eingerührt werden und dabei teilweise oder vollständig neutralisiert werden durch Zugabe einer basischen Substanz wie z. B. NaOH, KOH, Ammoniumhydroxyd oder organische Basen wie z. B. Triethanolamin. Es ist auch möglich ein Pulver des Copolymeren einzusetzen, dem bereits bei seiner Herstellung zum Zweck der (Teil)neutralisation eine Base z. B. NaOH zugesetzt wurde, so daß das Pulver ein bereits (teil)neutralisiertes Polymer ist. Der pH-Wert der Lösung liegt in der Regel über 4, z. B. im Bereich von 4 bis ca. 7. Man kann dabei auch z. B. Mischungen von Batches von voll- oder teilneutralisierten Dispersionen mit nicht neutralisierten Dispersionen vornehmen und in beschriebener Weise weiterverarbeiten, d. h. die Mischung für Überzüge verwenden oder zunächst zu einem Pulver gefrier- oder sprühtrocknen.

Die Dispersion kann z. B. auch in an sich bekannter Weise sprühgetrocknet oder gefriergetrocknet werden und im Form eines redispergierbaren Pulvers bereitgestellt werden (siehe z. B. EP-A 0 262 326). Alternative Verfahren sind die Gefriertrocknung oder Coagulation uns Abquetschen des Wassers in einem Extruder mit anschließender Granulation (siehe z. B. EP-A 0 683 028).

Überraschenderweise wurde gefunden, daß Copolymer-Dispersionen aus sprüh- oder gefriergetrockneten und redispergierten Pulvern eine erhöhte Scherstabilität aufweisen. Dies ist insbesondere beim Sprühauftrag von Vorteil. Dieser Vorteil tritt insbesondere verstärkt hervor wenn das in der Dispersion enthaltene Copolymer zu 2 bis 10, bevorzugt zu 5 bis 7 Mol-% in teilneutralisierter Form vorliegt (bezogen auf die im Copolymer enthaltenen Säuregruppen). Bevorzugt ist zu diesem Zweck die Teilneutalisation mittels Zugabe von NaOH. Bevorzugt ist ein anionischer Emulgator in Menge von 0,1 bis 2 Gew.-% enthalten. Besonders bevorzugt ist Natiumlaurylsulfat als Emulgator.

### "Ready to Use"

Das Copolymer kann in Pulverform in Mischung mit üblichen pharmazeutischen Zuschlagstoffen in einer leicht redispergierbaren Form vorliegen.

Geht man von einem Copolymer-Pulver, z. B einem bereits teilneutralisierten Pulver aus, so kann dieses trocken mit üblichen pharmazeutischen Zuschlagstoffen, wie z. B. Talkum, löslichen Farbstoffen, Farbstoffpigmenten oder Stabilisatoren formuliert bzw. gemischt und/oder vermahlen werden. Man spricht von einer "Ready to Use"-Formulierung, die nach Zusatz von Wasser und vergleichsweise kurzer Redispergierzeit direkt als fertiges oder zumindest weitgehend komplettes Überzugs- oder Bindemittel verwendet werden kann.

### Mechanische Eigenschaften

Im Bereich der erfiridungsgemäß ausgewählten Copolymerzusammensetzung wird ein sprunghafter Anstieg der mechanischen Belastbarkeit, d. h. insbesondere der Scherstabilität, festgestellt. Dabei ist die mechanische Belastbarkeit des Copolymers bereits ohne Weichmacher gegenüber nicht erfindungsgemäßen Copolymeren ähnlicher Zusammensetzung aber mit höherer Glastemperatur deutlich verbessert. Nicht erfindungsgemäße Copolymere mit ähnlicher Zusammensetzung und Glastemperaturen von 55 - 70 °C weisen zudem nicht mehr das gewünschte Freisetzungsprofil auf.

Die Unterschiede treten unter Zusatz von Weichmacher besonders deutlich hervor. Ab einem Gehalt von 1 Gew.-% Weichmacher sind bereits Unterschiede im Reißdehnungsverhalten (nach DIN 53 455) meßbar. Das Copolymer kann 0 bis 40 Gew.-% Weichmacher enthalten. Günstig sind in der Regel 6 bis 30, bevorzugt 15 bis 25 Gew.-% Weichmacher. Das weichmacherhaltige Copolymer kann Reißdehnungswerte [%] von mindestens 250, mindestens 300, mindestens 400, 250 bis 500 oder 300 bis 450 aufweisen.

### Polymermischungen

Zur Steuerung der Wirkstoffabgabe kann es im Einzelfall vorteilhaft sein, dem Copolymer weitere Polymere zuzumischen. Der Anteil weiterer Polymere an der Mischung kann in weiten Bereichen variieren und liegt zwischen 5 und 95, bevorzugt zwischen 10 und 90 Gew.-%, insbesondere bevorzugt zwischen 25 und 85 Gew.-%.

Beispiele für solche weiteren Polymere sind: Polyvinylpyrolidone, Polyvinylalkohole, anionische (Meth)acrylat-Copolymere aus Methylmethacrylat und/oder Ethylacrylat und Methacrylsäure (EUDRAGIT^{®} L 100, EUDRAGIT^{®} S 100, EUDRAGIT^{®} L 100-55). Anionische (Meth)acrylat-Copolymere aus Methylmethacrylat, Methylacrylat und Methacrylsäure des Standes der Technik (s. z. B. EP-A 0 704 207 oder EP-A 0 704 208), Carboxymethylcellulose-Salze, Hydroxypropylcellulose (HPMC), neutrale (Meth)acrylat Copolymere aus Methylmethacrylat und Ethylacrylat (Trockensubstanz aus EUDRAGIT^{®} NE 30 D), Copolymere aus Methylmethacrylat und Butylmethacrylat (PLASTOID^{®} B) oder (Meth)acrylat Copolymere mit quaternären Ammoniumgruppen (EUDRAGIT^{®} RL bzw. EUDRAGIT^{®} RS).

### Mehrschichtige Arzneiformen

Zur Steuerung der Wirkstoffabgabe kann es im Einzelfall vorteilhaft sein, die Arzneiform mit weiteren Polymer- oder Copolymerschichten zu versehen, die innerhalb oder außerhalb einer Schicht mit dem erfindungsgemäßen Copolymer angeordnet sein können. Beispielsweise kann eine äußere retardierende oder eine weitere äußere magensaftresistente und/oder geschmacksisolierende Schicht hinzugefügt werden. Ein weiteres Beispiel hierfür wäre das Aufbringen einer äußeren gefärbten Polymerschicht, z. B. aus Hydroxypropylmethylcellulose (HPMC) und Farbstoffen.

Beispiele für solche weiteren Polymere sind: Polyvinylpyrolidone, Polyvinylalkohole, anionische (Meth)acrylat-Copolymere aus Methylmethacrylat und/oder Ethylacrylat und Methacrylsäure (EUDRAGIT^{®} L 100, EUDRAGIT^{®} S 100, EUDRAGIT^{®} L 100-55). Anionische (Meth)acrylat-Copolymere aus Methylmethacrylat, Methylacrylat und Methacrylsäure des Standes der Technik (s. z. B. EP-A 0 704 207 oder EP-A 0 704 208), Carboxymethylcellulose-Salze, Hydroxypropylmethylcellulose (HPMC), neutrale (Meth)acrylat Copolymere aus Methylmethacrylat und Ethylacrylat (Trockensubstanz aus EUDRAGIT^{®} NE 30 D), Copolymere aus Methylmethacrylat und Butylmethacrylat (PLASTOID^{®} B) oder (Meth)acrylat Copolymere mit quaternären Ammoniumgruppen (EUDRAGIT^{®} RL bzw. EUDRAGIT^{®} RS).

### Arzneiform

Die erfindungsgemäße Arzneiform liegt als Tablette, Pellets enthaltende Arzneiform, und/oder in Form einer wirkstoffhaltigen Matrix, wobei die Tabletten, Pellets, und/oder die wirkstoffhaltige Matrix einen pharmazeutischen Wirkstoff und ein Copolymer als Überzugs- und/oder Bindemittel, sowie gegebenenfalls einen Kern und pharmazeutisch übliche Zuschlagstoffe enthalten. Tabletten bestehen (Filmtabletten) üblicherweise aus mit einem Copolymer überzogenen wirkstoffhaltigen Kernen. Pellets bestehen meist aus überzogenen Kernen oder auch aus überzogenen Wirkstoffkristallen. In einer wirkstoffhaltige Matrix fungiert das Copolymer als Bindemittel für den Wirkstoff.

Bei der Begriff "Arzneiform als Tablette, Pellets enthaltende Arzneiform, und/oder in Form einer wirkstoffhaltigen Matrix" soll sinngemäß alle gängige Typen von Arzneiformen beinhalten, die den Fachmann bekannt sind. Insbesondere handelt es sich um Tabletten, Tabletten mit verzögertem oder beschleunigtem Zerfall, Minitabletten, Pellets, auch im Sinne von Granulaten, Mikropartikeln oder Mikrotabletten zu verstehen, zu Pellets verpreßte Tabletten (multipartikuläre Arzneiform), in Kapseln verfüllte Pellets, Minitabletten oder Granulate. Transdermale Therapiesysteme z. B. in Form eines Pflasters oder eines Ausstrichs sind Beispiele für wirkstoffhaltige Matrices. Weiterhin kann es sich um Kapseln, Teile von Kapseln oder sonstigen Arzneiformen, Sachets, Trockensäfte, Suppositorien, Vaginalia oder Implantate handeln.

Nach dem erfindungsgemäßen Verfahren kann man das Copolymer gegebenenfalls in Kombination mit pharmazeutisch üblichen Zusatzstoffen, z. B. Weichmachern, Formtrennmitteln und/oder Farbstoffen, auch zunächst zu Formkörpern verarbeiten und anschließend darin einen pharmazeutischen Wirkstoff mit oder ohne Kern einschließen. Diese Verarbeitung kann bevorzugt durch Tauchen, Spritzgießen oder Extrudieren erfolgen. Der Formkörper kann eine Kapsel, der Teil einer Kapsel oder eine schweißbare Folie sein.

### Herstellung der Arzneiform

Die Arzneiform wird hergestellt, indem man das Copolymer, den pharmazeutischen Wirkstoffs, den gegebenenfalls vorhandenen Kern und/oder die pharmazeutisch üblichen Zuschlagstoffe in an sich bekannter Weise, mit oder ohne Zusatz von Wasser, durch Schmelzen, Spritzguß, Extrusion, Feuchtgranulieren, Gießen, Ausstreichen, Aufsprühen oder Verpressen zu Tabletten oder Pellets enthaltenden Arzneiformen und/oder einer wirkstoffhaltigen Matrix verarbeitet.

### Weitere Verwendungen

Außer für Arzneiformen kann das erfindungsgemäße Copolymer auch als Bestandteil oder Inhaltsstoff von Kosmetika oder Nahrungsergänzungsstoffen verwendet werden. Auf dem Gebiet der Kosmetika kann das Copolymer bevorzugt in gelöster Form z. B. in Salben und Cremes oder als Bestandteil kosmetischer Pflaster eingearbeitet werden. Bei den Nahrungsergänzungsstoffen kann da Copolymer z. B. zur Geschmacksisolierung, als Umhüllung zum Schutz von Vitaminen oder Mineralstoffen sowie zur Isolierung unverträglicher Bestandteile verwendet werden.

### Wirkstoffe

Die im Sinne der Erfindung eingesetzten Wirkstoffe sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen. Erfindungsgemäß können alle Wirkstoffe eingesetzt werden, die die gewünschte therapeutische Wirkung im Sinne der obigen Definition erfüllen und eine ausreichende thermische Stabilität besitzen.

Die erfindungsgemäße Formulierung eignet sich zur Verabreichung grundsätzlich beliebiger pharmazeutischer Wirkstoffe, die vorzugsweise im Darm und/oder Colon freigesetzt werden sollen, und insbesondere solcher, die mit Vorteil in retardierter Form verabreicht werden können.

Insbesondere sind Wirkstoff aus den folgenden Wirkstoffklasse zu nennen: Abführmittel, Analgetika, Antibiotika, Antirheumatika, Antiallergika, Antiarrhythmika, Antibiotika, Antiepileptica, Betarezeptorenblocker, Calciumkanalblocker, Chemotherapeutika, Enzyme, Extrakte, Hemmstoffe des Renin-Angiotensin-Systems, Broncholytika/Antiasthmatika Cholinergika, Diuretika, Durchblutungsfördernde Mittel, Gichtmittel Grippemittel, Koronarmittel, Osteoporosemittel (Biphosphonate), Lipidsenker, Magen-Darmmittel, Peptide, Proteine, Protonenpumpenblocker, Psychopharmaka" Thrombozytenaggregationshemmer Urologika Venetherapeutika, Vitamine und Mineralien.

Die erfindungsgemäße Arzneiform kann z. B. folgende Wirkstoffe enthalten: gekennzeichnet, daß als Wirkstoff Paroxetin, Reboxetin Morphin und dessen Derivate,Tramadol, Bisacodyl, Natriumfluorid Acamprosat-Ca, Digitoxin, Dimethicon. Kolibakterien, Liponsäure, Methenamin, Budenosid, Acetylsalicylsäure, Diclofenac, Flurbiprophen, Indometacin, Lonazolac, Hydrocortison, Ibuprofen, Ketoprofen,Prdnisolon, Propyphenazon, Naproxen, Paracetamol, Flurbiprofen, Dimetinden, Chinidin, Metoprolol, Propranolol, Oxprenolol, Pindolol, Atenolol, Metoprolol, Disopyramid, Verapamil, Diltiazem, Gallopamil, Nifedipin, Nicardipin, Nisoldipin, Nimodipin, Amlodipin, Theophyllin, Salbutamol, Terbutalin, Ambroxol, Aminophyllin, Carbamazepin, Alendronat, Etidronat, Clodronat, Pamidronat, Ibandronate Cholintheophyllinat, Pyridostigmin, Piretanid, Furosemid, Pentoxifyllin, Naftidrofuryl, Buflomedil, Xantinolnicotinat, Bencyclan, Allopurinol, Norephedrin, Clorphenamin Isosorbidmononitrat, Isosorbiddinitrat, Glyceroltrinitrat, Molsidomin, Bezafibrat, Fenofibrat, Gemfibrozil, Cerivastatin, Pravastatin, Fluvastatin, Lovastatin, Atorvastatin, Simvastatin, 5-Aminosalicylsäure, Sulfasalazin, Budenosid, Natamycin, Preglumetacin Sulfasalacin, Nitrofurantion Xantinol, Metoclopramid, Amitriptylin, Dibenzepin, Venlafaxin, Thioridazin, Oxazepam, Omeprazol, Lanzoprazol, Pantoprazol, Rabeprazol, Perprazol, Esomprazol, , Nitrofurantoin, Rutosid, Knoblauch, Aescin,Bromelain, Pankreatin oder Trypsin, ein Insulin, ein Human Growth Hormon (hGH), Corbaplatin, Intron A, Calcitonin, Cromalyn, ein Interferon, ein Calcitonin, Granulocyte Colony Stimulating factor (G-CSF), ein Interleukin, ein Kinin, Parathyroidhormone, Glucagon, Pindolol, ProSomatostatin, ein Somatostatin, Detirelix, Cetrorelix, Vasopressin, 1-Deaminocysteine-8-D-arginine-Vasopressin, Leuprolidacetat oder ein Antigen, das aus Gräsern oder anderen Pflanzen, wie z. B. Roggen, Weizen, Gerste, Hafer, Bermuda Gras, Zinnkraut, Ahorn, Ulme, Eiche, Platane, Pappel, Zeder, Zinnkraut, Disteln gewonnen wurde, IgG, spezifische Impfstoffe oder monoclonale Antikörper, pflanzliche Trockenextrakt, Ascorbinsäure, Aspartamsäure, Valproinsäure Zink, und Kalium, Natrium, Lithium und deren pharmazeutisch verwendete Salze.

Die Wirkstoffe können gewünschtenfalls auch in Form ihrer pharmazeutisch annehmbaren Salze oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemässen Zusammensetzungen auch zwei oder mehrere pharmazeutische Wirkstoffe enthalten.

### Pharmazeutisch übliche Zuschlagstoffe

### a) Weichmacher

Als Weichmacher geeignete Stoffe haben in der Regel ein Molekulargewicht zwischen 100 und 20 000 und enthalten eine oder mehrere hydrophile Gruppen im Molekül, z. B. Hydroxyl- , Ester- oder Aminogruppen. Geeignet sind Citrate, Phthalate, Sebacate, Rizinusöl. Beispiele geeigneter Weichmacher sind Citronensäurealkylester, Glycerinester, Phtalsäurealkylester, Sebacinsäurealkylester, Sucroseester, Sorbitanester, und Polyethylenglykole 4000 bis 20.000. Bevorzugte Weichmacher sind Tributylcitrat, Triethylcitrat, Acetyltriethylcitrat, Dibutylsebacat und Diethylsebacat. Bevorzugt setzt man dem Copolymer bis zu 30, insbesondere 5 bis 25 Gew.-% eines Weichmachers, bezogen auf das Trockengewicht des Copolymers, zu

### b) Weitere pharmazeutisch übliche Zuschlagstoffe

Hier sind z. B, Stabilisatoren, Farbstoffe, Antioxidantien, Netzmittel, Pigmente, Glanzmittel etc. zu nennen. Sie dienen vor allem als Verarbeitungshilfsmittel und sollen ein sicheres und reproduzierbares Herstellungsverfahren sowie gute Langzeitlagerstabilität gewährleisten werden kann. Pharmazeutisch übliche Hilfsstoffe können in Mengen von 0,001 Gew-% bis 300 Gew.-%, bevorzugt 0,1 bis 100 Gew.-% bezogen auf das Copolymere vorliegen.

Beispiele für Trockenstellmittel sind: Aluminiumoxid, Magnesiumoxid, Kaolin, Talkum, Kieselsäure (Aerosile), Bariumsulfat, Ruß und Cellulose.

Im Gegensatz zu Trockenstellmitteln haben Formtrennmittel die Eigenschaft, die Klebkraft zwischen dem Formteilen und einer Geräteoberfläche, in dem die Arzneiform hergestellt wird, zu reduzieren. Dadurch wird es möglich, Formteile herzustellen, die nicht zerbrochen und geometrisch nicht deformiert sind. Formtrennmittel sind meist teilverträglich oder unverträglich mit den Polymeren, in denen sie besonders wirksam sind. Durch die Teil- bzw. Unverträglichkeit tritt beim Einspritzen der Schmelze in den Formhohlraum eine Migration in die Grenzfläche des Überganges zwischen Werkzeugwandung und Formteil auf. Damit Formtrennmittel besonders vorteilhaft migrieren können, muss der Schmelzpunkt des Formtrennmittels 20°C bis 100°C unterhalb der Verarbeitungstemperatur des Polymeren liegen.

Beispiele für Trennmittel (Formtrennmittel) sind: Ester von Fettsäuren oder Fettsäureamide , aliphatische, langkettige Carbonsäuren, Fettalkohole sowie deren Ester, Montan- oder Paraffinwachse und Metallseifen, insbesondere zu nennen sind Glycerolmonostearat, Stearylalkohol, Glycerolbehensäureester, Cetylalkohol, Palmitinsäure, Kanaubawachs, Bienenwachs etc..

### Herstellung multipartikulärer Arzneiformen

Die Erfindung eignet sich insbesondere zur Herstellung multipartikulärer Arzneiformen, da das erfindungsgemäße Copolymer den hohen Drücken beim Verpressen der Pellets mit dem Füllstoff standhält.

Die Herstellung von multipartikulären Arzneiformen durch Verpressen eines pharmazeutisch üblichen Bindemittels mit wirkstoffhaltigen Partikeln ist z. B. Beckert et al. (1996), "Compression of enteric-coated pellets to disintegrating tabtets", International Journal of Pharmaceutics 143, S. 13 - 23*,* und in WO 96/01624 ausführlich beschrieben.

Wirkstoffhaltige Pellets können hergestellt werden indem man mittels eines Layeringprozesses Wirkstoff aufbringt. Dazu wird Wirkstoff gemeinsam mit weiteren Hilfsstoffen (Trennmittel, ggf. Weichmacher) homogenisiert und in einem Bindemittel gelöst oder suspendiert. Mittels eines Wirbelschichtverfahrens kann die Flüssigkeit auf Placebopellets oder sonstige geeignete Trägermaterialien aufgebracht werden, wobei das Lösungs- oder Suspendiermittel verdunstet wird (Literatur: International Journal of Pharmaceutics 143, S. 13 - 23). Nach dem Herstellverfahren kann sich ein Trocknungsschritt anschließen. Der Wirkstoff kann in mehreren Schichten aufgebracht werden.

Einige Wirkstoffe, z. B. Acetylsalicylsäure, sind in Form von Wirkstoffkristallen handelsüblich und können in dieser Form anstelle von wirkstoffhaltigen Pellets eingesetzt werden.

Filmüberzüge auf wirkstoffhaltige Pellets werden üblicherweise in Wirbelschichtgeräten aufgebracht. Rezepturbeispiele sind in dieser Anmeldung erwähnt. Filmbildner werden üblicherweise mit Weichmachern und Trennmitteln nach einem geeigneten Verfahren gemischt. Hierbei können die Filmbildner als Lösung oder Suspension vorliegen. Die Hilfsstoffe für die Filmbildung können ebenfalls gelöst oder suspendiert sein. Organische oder wässrige Löse- oder Dispergiermittel können verwendet werden. Zur Stabilisierung der Dispersion können zusätzlich Stabilisatoren verwendet werden (Beispiel: Tween 80 oder andere geeignete Emulgatoren bzw. Stabilisatoren).

Beispiele für Trennmittel sind Glycerolmonostearat oder andere geeignete Fettsäurederivate, Kieselsäurederivate oder Talkum. Beispiele für Weichmacher sind Propylenglykol, Phthalate, Polyethylenglykole, Sebacate oder Citrate, sowie andere in der Literatur erwähnte Substanzen.

Zwischen wirkstoffhaltiger und darmlöslicher Copolymer-Schicht kann eine trennende Schicht aufgebracht sein, die der Trennung von Wirkstoff und Überzugsmaterial zum Zwecke der Verhinderung von Interaktionen dient. Diese Schicht kann aus inerten Filmbildnern (z.B. HPMC, HPC oder (Meth)acrylsäure-Copolymeren) oder z.B. Talkum oder anderen geeigneten pharmazeutischen Substanzen bestehen. Ebenso können Kombinationen aus Filmbildnern und Talkum oder ähnlichen Stoffen verwendet werden.

Es ist auch möglich eine Trennschicht aus teilweise bzw. vollneutralisierten Copolymer-Dispersionen aufzubringen.

Mischungen zur Herstellung von Tabletten aus überzogenen Partikeln werden durch Vermischen der Pellets mit geeigneten Bindemitteln für die Tablettierung, nötigenfalls der Zugabe von zerfallsfördernden Substanzen und nötigenfalls der Zugabe von Schmiermitteln zubereitet. Das Mischen kann in geeigneten Maschinen stattfinden. Ungeeignet sind Mischer, die zu Schäden an den überzogenen Partikeln führen, z. B. Pflugscharmischer. Zur Erzielung geeigneter kurzer Zerfallszeiten kann eine spezielle Reihenfolge bei der Zugabe der Hilfsstoffe zu den überzogenen Partikel erforderlich sein. Durch Vormischung mit der überzogenen Partikel mit dem Schmier- oder Formentrennmittel Magnesiumstearat kann dessen Oberfläche hydrophobisiert und somit Verkleben vermieden werden.

Zum Tablettieren geeignete Mischungen enthalten üblicherweise 3 bis 15 Gew.-% eines Zerfallshilfsmittels, z. B. Kollidon CL und z. B. 0,1 bis 1 Gew.-% eines Schmier- und Formentrennmittels wie Magnesiumstearat. Der Bindemittelanteil bestimmt sich nach dem geforderten Anteil an überzogenen Partikeln.

Typische Bindemittel sind z. B. Cellactose^{®}, mikrokristalline Cellulose, Calciumphosphate, Ludipress^{®}, Lactose oder andere geeignete Zucker, Calciumsulfate oder Stärkederivate. Bevorzugt werden Substanzen mit geringer Schüttdichte.

Typische Zerfallshilfsmittel (Sprengmittel) sind quervernetzte Stärke- oder Cellulosederivate, sowie quervernetztes Polyvinylpyrrolidon. Ebenso sind Cellulosederivate geeignet. Durch Auswahl eines geeigneten Bindemittels kann die Verwendung von Zerfallshilfsmittel entfallen.

Typische Schmier- und Formentrennmittel sind Magnesiumstearate oder andere geeignete Salze von Fettsäuren oder in der Literatur zu diesem Zweck aufgeführte Substanzen (z.B. Laurinsäure, Calciumstearat, Talkum usw.). Bei Verwendung geeigneter Maschinen (z.B. Tablettenpresse mit externer Schmierung) oder geeigneter Formulierungen kann die Verwendung eines Schmier- und Formentrennmittels in der Mischung entfallen.

Der Mischung kann gegebenenfalls ein Hilfsmittel zur Fließverbesserung beigefügt sein (z. B. hochdisperse Kieselsäurederivate, Talkum usw.).

Das Tablettieren kann auf üblichen Tablettenpressen, Exzenter- oder Rundlauftablettenpressen erfolgen, bei Preßkräften im Bereich von 5 bis 40 kN, bevorzugt 10-20 kN. Die Tablettenpressen können mit Systemen zur externen Schmierung ausgestattet sein. Gegebenenfalls kommen spezielle Systeme zur Matrizenbefüllung zum Einsatz, die die Matrizenbefüllung mittels Rührflügein vermeiden.

### Weitere Herstellungsverfahren für die erfindungsgemäße Arzneiform

Auftragsverfahren erfolgt mittels Sprühauftrag aus organischer Lösung, oder bevorzugt wäßrigen Dispersionen durch Schmelzen oder durch direkten Pulverauftrag. Für die Ausführung ist dabei entscheidend, daß gleichmäßige, porenfreie Überzüge entstehen.

Auftragsverfahren gemäß Stand der Technik s. z.B. Bauer, Lehmann, Osterwald, Rothgang, "Überzogene Arzneiformen" Wissenschaftliche Verlagsgesellschaft mbH Stuttgart, Kap 7,S.165 - 196

Für die Applikation sind relevante Eigenschaften, geforderte Tests und Spezifikationen in Arzneibüchern aufgelistet.

Details sind den gängigen Lehrbüchern zu entnehmen, z.B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P. : Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

### Vorteilhafte Wirkungen der Erfindung

Eine schnelle Wirkstofffreigabe bei gleichzeitiger Isolierung des Wirkstoffs, Geschmacks- und/oder Geruchsschutz kann erreicht werden, z. B. in dünn umhüllten Arzneiformen oder Kosmetika oder Nahrungsergänzungsstoffen. In diesem Fall liegen die Schichtdicken z. B. im Bereich von 1 bis 15 µm.

Eine leicht verzögerte Wirkstoffabgabe in Magen und Darm kann erreicht werden z. B. in umhüllten Arzneiformen oder Kosmetika oder Nahrungsergänzungsstoffen. In diesem Fall liegen die Schichtdicken z. B. im Bereich von 15 bis 40 µm. Die Auflösung des Copolymerfilms bzw. der Copolymermatrix erfolgt günstigerweise ab etwa pH 6,0.

Magensaftresistenz und gegebenenfalls Bioverfügbarkeitssteigerung kann erreicht werden z. B. in umhüllten Arzneiformen oder Kosmetika oder Nahrungsergänzungsstoffen. In diesem Fall liegen die Schichtdicken z. B. im Bereich von 40 bis 60 µm.

Wirkstofffreigabe in tieferen Darmabschnitten und gegebenenfalls Bioverfügbarkeitssteigerung kann erreicht werden z. B. in umhüllten Arzneiformen oder Kosmetika oder Nahrungsergänzungsstoffen. In diesem Fall liegen die Schichtdicken z. B. im Bereich von 70 bis 100 µm.

Eine beschleunigte Freigabe bei gleichzeitiger Steigerung der Bioverfügbarkeit kann erreicht werden aus Matrixsystemen, in denen der Copolymeranteil in Gew.-% größer ist als der Wirkstoffanteil.

Copolymerfilme weisen eine hohe Reißdehnung auf und sind daher ohne oder schon mit geringem Weichmacherzusatz ausreichend elastisch. Es lassen sich Copolymer-Dispersionen herstellen, die keine oder nur sehr geringe Neigung zur Coagulat-Bildung aufweisen. Die Dispersionen sind daher sehr verarbeitungssicher und lagerstabil.

### BEISPIELE

### Beispiel 1: Eigenschaftsvergleich erfindungsqemäßer und nicht erfindungsgemäßer Copolymerisate

| **Copolymer** | **A** | **B** | **C** | *V1* | *V2* | *V3* | *V4* | *V5 (L30D- 55)* | *V6 (FS30D)* |
|---|---|---|---|---|---|---|---|---|---|
| **Methacrylsäure** | **30** | **30** | **30** | ***30*** | ***30*** | *35* | ***30*** | *50* | *10* |
| **Methylacrylat** | **25** | **20** | **15** | ***10*** | ***40*** | ***18*** | ***30*** | - | ***65*** |
| **Ethylacrylat** | **30** | **30** | **30** | ***30*** | ***30*** | ***29*** | ***30*** | *50* | - |
| **Butylmethacrylat** | **15** | **20** | **25** | ***30*** | - | ***18*** | *10* | - | - |
| **Methylmethacrylat** | - | - | - | - | - | - | - | - | *35* |
| **Glastemperatur Tg °C [DIN 11357, 3.3.3]** | **56** | **65** | **67** | *73* | ***56*** | ***65*** | ***61*** | *115* | *48* |
| **Reißdehnung[%**] **20 Gew.% TEC DIN 53455** | **423** | **296** | **169** | *133* | **320** | *77* | ***362*** | *10* | ***1000*** |
| **Coagulat-Bildung in der Dispersion nach** | | | | | | | | | |
| **3h** | **0** | **0** | **0** | ***0*** | *1* | **0** | *1* | *0* | *0* |
| **6h** | **1** | **0** | **0** | ***0*** | *2* | **0** | *1* | *0* | *0* |
| **24h** | **1** | **0** | **0** | ***0*** | *3* | **0** | *2* | *0* | *1* |

Die Reißdehnung [%] wurde nach DIN 53455 unter Zusatz von 20 Gew.-% Triethylcitrat (TEC) bestimmt. Die Coagulat-Bildung in der Dispersion wurde in einem Rührtest mit Magnetrührer bei Raumtemperatur und einer Rührerdrehzahl von ca. 100 pro Minute bestimmt.

Die Ergebnisse wurden nach 3, 6 und 24 h wie folgt bewertet:
0 = kein Coagulat (Anforderung für den 3 und 6h-Wert)
1 = leichte Coagulat-Bildung im Sieb (beim 3 und 6h-Wert nicht akzeptabel)
2 = viel Koagulat im Sieb (nicht akzeptabel)
3 = starke Coagulat-Bildung im Sieb und auf dem Magnetrührer (nicht akzeptabel)

Die Copolymere A bis C stellen erfindungsgemäße Copolymere dar.

Die Copolymere A bis C zeichnen sich durch gute (A und B) bis gerade noch akzeptable (C) Reißdehnungswerte aus. Die Glastemperatur in Bereich von 55 bis 70 °C bewirkt, daß die Polymere gute mechanische Stabilität aufweisen.

Die Copolymer V1 bis V6 stellen nicht erfindungsgemäße Copolymere dar.

Beim Copolymer V1 liegt zwar eine Monomerzusammensetzung innerhalb der erfindungsgemäßen Anteile vor, die Anteile sind jedoch so gewählt, daß die anspruchsgemäße Maßgabe der Obergrenze der Glastemperatur von 70 °C überschritten wird. Das Copolymer ist in Folge zu hart, die Reißdehnung ist unbefriedigend.

Beim Copolymer V2 ist das Monomer Butylmethacrylat nicht enthalten. Die Abwesenheit dieses Monomeren bedingt eine Neigung zur Coagulation resultierender Dispersionen.

Beim Copolymer V3 ist der anspruchsgemäß geforderte Anteil für Methacrylsäure überschritten. Die Folge ist ein deutlicher Abfall der Reißdehnung.

Beim Copolymer V4 liegt der Anteil an Butylmethacrylat knapp außerhalb der geforderten Grenze. Es ist eine leichte Neigung zur Coagulat-Bildung vorhanden, wodurch die gewünschte Verarbeitungssicherheit nicht mehr als gegeben angesehen wird.

Das Copolymer V5 (EUDRAGIT^{®} L30D-55) enthält kein Methylmethacrylat sowie kein Butylmethacrylat und hat eine sehr geringe Reißdehnung bei zugleich sehr hoher Glastemperatur. Das Copolymer V5 erfordert deshalb hohe Weichmacherzusätze, deutlich über 20 Gew.-%, um akzeptable Reißdehnungswerte zu erreichen. Dabei besteht jedoch immer die Möglichkeit, daß die Eigenschaften des Filmüberzugs in unerwünschter Weise beeinflußt werden. In Einzelfällen können z. B. Entmischungserscheinungen nach Lagerung auftreten.

Das Copolymer V6 (EUDRAGIT^{®} FS30D) hat einen geringen Methacrylsäure-Gehalt und enthält kein Ethylacrylat oder Butylmethacrylat. Die Glastemperatur ist vergleichsweise niedrig. Das Copolymer V6 besitzt ein extrem gutes Reißdehnungsverhalten. Dieses Polymer löst sich jedoch erst oberhalb pH 7,0 auf und besitzt somit ein völlig anderes Freigabeprofil als die erfindungsgemäßen Copolymere, die sich ab etwa pH 6,0 aufzulösen beginnen.

### Beispiel 2 (Überzug auf Chinidinsulfattabletten)

### Aus

| | |
|---|---|
| Emulsionspolymerisat Copolymer B* | 469,7 g |
| Glycerolmonostearat (GMS) | 7,0 g |
| Polysorbat 80 (33%ige wässrige Lösung) | 8,5 g |
| Wasser, gereinigt | 268,7 g |

| | |
|---|---|
| *(Copolymer B = Copolymer aus 30 Gew.% Methacrylsäure, 20 Gew.-% Methylacrylat, 30 Gew.-% Methylacrylat und 20 Gew.-% Butylmethacrylat) | |

wird eine Sprühsuspension hergestellt, indem GMS und Polysorbat 80 bei 65°C im Wasser emulgiert und unter Rühren auf Raumtemperatur abgekühlt wird. In diese Emulsion wird das Emulsionspolymerisat eingerührt.

In einem 35 cm Dragierkessel (Durchmesser 35 cm) mit Antrieb AR 400 (Fa. Erweka, Heusenstamm) und einer Sprühpistole (Fa. Schlick, Mod. 970 Form 7-1 S 21) wird eine Mischung aus 2300 g Placebokernen (10 mm Durchmesser, 300 mg Gewicht) und 200 g Chinidinsulfattabletten (5 % Wirkstoffgehalt, 10 mm Durchmesser, 300 mg Gewicht) unter folgenden Bedingungen überzogen:

| | |
|---|---|
| Düsendurchmesser | 1,2 mm |
| Kesseldrehzahl | 40 U/min |
| Zulufttemperatur | 30 - 42°C |
| Produkttemperatur | 28 - 30°C |
| Sprühdruck | 0,8 bar |
| Sprühgeschwindigkeit | 8 - 9,5 g/min |
| Nachtrocknung | 2 Std. bei 40°C auf Horden |

Der Polymerauftrag betrug 6,0 mg/cm².

Die überzogenen Chinidinsulfattabletten zeigten im Dissolutiontest nach Pharm. Eur. folgende Werte:

| Zeit [min] | pH | Freigabe [%] |
|---|---|---|
| 10 | 1,2 | < 1,0 |
| 60 | 1,2 | < 1,0 |
| 120 | 1,2 | < 1,0 |
| 140 | 6,8 | 20 |
| 150 | 6,8 | 84 |
| 180 | 6,8 | 100 |

### Beispiel 3 (Überzug auf Bisacodylpellets)

Aus

| | |
|---|---|
| Emulsionspolymerisat Copolymer B | 53,3 g |
| Glycerolmonostearat (GMS) | 1,6 g |
| Polysorbat 80 (33%ige wässrige Lösung) | 1,9 g |
| Wasser, gereinigt | 64,7 g |

wird eine Sprühsuspension hergestellt, indem GMS und Polysorbat 80 bei 65°C im Wasser emulgiert und unter Rühren auf Raumtemperatur abgekühlt wird. In diese Emulsion wird das Emulsionspolymerisat eingerührt.

In einem Wirbelschichtgerät (Typ: Mini Glatt, Fa. GLATT) werden mittels Bottom-Spray 200 g Bisacodylpellets unter folgenden Bedingungen überzogen:

| | |
|---|---|
| Düsendurchmesser | 0,5 mm |
| Zulufttemperatur | 35°C |
| Produkttemperatur | 31 - 32,5°C |
| Sprühdruck | 0,5 bar |
| Sprühgeschwindigkeit | 1,6 -1,8 g/min |
| Nachtrocknung | 2 Std. bei 40°C auf Horden |

Der Polymerauftrag betrug 8 %.

Die überzogenen Bisacodylpellets zeigten im Dissolutiontest nach Pharm. Eur. folgende Werte:

| Zeit [min] | pH | Freigabe [%] |
|---|---|---|
| 15 | 1,2 | < 1,0 |
| 60 | 1,2 | < 1,0 |
| 120 | 1,2 | 1,3 |
| 180 | 6,8 | 99,7 |

### Beispiel 4 (Überzug auf Bisacodylpellets)

Aus

| | |
|---|---|
| Emulsionspolymerisat Copolymer B | 65,0 g |
| Glycerolmonostearat (GMS) | 1,95 g |
| Polysorbat 80 (33%ige wässrige Lösung) | 2,36 g |
| Wasser, gereinigt | 78,9 g |

wird eine Sprühsuspension hergestellt, indem GMS und Polysorbat 80 bei 65°C im Wasser emulgiert und unter Rühren auf Raumtemperatur abgekühlt wird. In diese Emulsion wird das Emulsionspolymerisat eingerührt.

In einem Wirbelschichtgerät (Typ: Mini Glatt, Fa. GLATT) werden mittels Bottom-Spray 150 g Bisacodylpellets unter folgenden Bedingungen überzogen:

| | |
|---|---|
| Düsendurchmesser | 0,5 mm |
| Zulufttemperatur | 35°C |
| Produkttemperatur | 31 - 32,5°C |
| Sprühdruck | 0,5 bar |
| Sprühgeschwindigkeit | 1,6 - 1,8 g/min |
| Nachtrocknung | 2 Std. bei 40°C auf Horden |

Der Polymerauftrag betrug 13 %.

Die überzogenen Bisacodylpellets zeigten im Dissolutiontest nach Pharm. Eur. folgende Werte:

| Zeit [min] | pH | Freigabe [%] |
|---|---|---|
| 15 | 1,2 | < 1,0 |
| 60 | 1,2 | < 1,0 |
| 120 | 1,2 | < 1,0 |
| 180 | 6,8 | 97,0 |

### Beispiel 5 (Überzug auf Coffeinpellets)

Aus

| | |
|---|---|
| Emulsionspolymerisat Copolymer B | 266,7 g |
| Glycerolmonostearat (GMS) | 8,0 g |
| Triethylcitrat | 8,0 g |
| Polysorbat 80 (33%ige wässrige Lösung) | 9,7 g |
| Wasser, gereinigt | 203,6 g |

wird eine Sprühsuspension hergestellt, indem GMS, Triethylcitrat und Polysorbat 80 bei 65°C im Wasser emulgiert und unter Rühren auf Raumtemperatur abgekühlt wird. In diese Emulsion wird das Emulsionspolymerisat eingerührt.

In einem Wirbelschichtgerät (Typ: GPCG 1, Fa. GLATT) werden mittels Top-Spray 800 g Bisacodylpellets unter folgenden Bedingungen überzogen:

| | |
|---|---|
| Düsendurchmesser | 1,2 mm |
| Zulufttemperatur | 39°C |
| Produkttemperatur | 30°C |
| Sprühdruck | 1,8 bar |
| Sprühgeschwindigkeit | 12,5 g/min |
| Nachtrocknung | 2 Std. bei 40°C auf Horden |

Der Polymerauftrag betrug 10 %.

Die überzogenen Coffeinpellets zeigten im Dissolutiontest nach Pharm. Eur. folgende Werte:

| Zeit [min] | pH | Freigabe [%] |
|---|---|---|
| 15 | 1,2 | < 1,0 |
| 60 | 1,2 | 1,7 |
| 90 | 1,2 | 2,3 |
| 120 | 1,2 | 3,9 |
| 150 | 6,8 | 87,6 |
| 180 | 6,8 | 99,5 |

## Patentansprüche

1. Verfahren zur Herstellung einer überzogenen Arzneiform oder einer Arzneiform in Form einer wirkstoffhaltigen Matrix, indem man ein Copolymer, einen pharmazeutischen Wirkstoff, einen gegebenenfalls vorhandenen Kern und/oder pharmazeutisch übliche Zuschlagstoffe in an sich bekannter Weise durch Schmelzen, Spritzguß, Extrusion, Feuchtgranulieren, Gießen, Tauchen, Ausstreichen, Aufsprühen oder Verpressen zu einer überzogenen Arzneiform und/oder zu einer wirkstoffhaltigen Matrix verarbeitet,
**dadurch gekennzeichnet, daß** man ein Copolymer einsetzt, welches sich aus
20 bis 33 Gew.-% Methacrylsäure und/oder Acrylsäure,
5 bis 30 Gew.-% Methylacrylat und
20 bis 40 Gew.-% Ethylacrylat und
größer 10 bis 30 Gew.-% Butylmethacrylat und
gegebenenfalls
0 bis 10 Gew.-% weiteren vinylisch copolymerisierbarer Monomeren,
wobei sich die Anteile der Monomeren zu 100 Gew.-% addieren,
zusammensetzt, mit der Maßgabe, daß die Glastemperatur des Copolymers 55 bis 70 °C beträgt.

2. Verfahren nach Anspruch 1, **dadurch gekennzeichnet daß** man dem Überzugs- und Bindemittel 5 bis 25 Gew.-% eines Weichmachers zusetzt.

3. Verfahren nach Anspruch 1 oder 2, **dadurch gekennzeichnet, daß** zur Herstellung der Arzneiform das Copolymer in Form einer Dispersion verwendet wird.

4. Verfahren nach Anspruch 3, **dadurch gekennzeichnet, daß** die Dispersion durch Redispergierung eines gefriergetrockneten oder sprühgetrockneten Copolymer-Pulvers erhalten wurde.

5. Verfahren nach Anspruch 3 oder 4, **dadurch gekennzeichnet, daß** das in der Dispersion enthaltene Copolymer zu 2 bis 10 Gew.-% in teilneutralisierter Form vorliegt.

6. Verfahren nach Anspruch 5, **dadurch gekennzeichnet, daß** die Teilneutalisation mittels Zugabe von NaOH bewirkt wurde.

7. Verfahren nach einem oder mehreren der Ansprüche 3 bis 6, **dadurch gekennzeichnet, daß** ein anionischer Emulgator in Menge von 0,1 bis 2 Gew.-% enthalten ist.

8. Verfahren nach Anspruch 7, **dadurch gekennzeichnet, daß** Natiumlaurylsulfat als Emulgator enthalten ist.

9. Verfahren nach einem oder mehreren der Ansprüche 1 bis 8, **dadurch gekennzeichnet, daß** man das Copolymer gegebenenfalls in Kombination mit pharmazeutisch üblichen Zusatzstoffen zu Formkörpern verarbeitet und darin einen pharmazeutischen Wirkstoff einschließt.

10. Verfahren nach Anspruch 9, **dadurch gekennzeichnet, daß** der Formkörper eine Kapsel, der Teil einer Kapsel oder eine schweißbare Folie ist.

11. Arzneiform, herstellbar nach einem oder mehreren der Ansprüche 1 bis 10.

12. Arzneiform nach Anspruch 11, **dadurch gekennzeichnet, daß** es sich um Tabletten, Tabletten mit verzögertem oder beschleunigtem Zerfall, Minitabletten, Pellets, aus Pellets verpreßten Tabletten, in Kapseln verfüllte Pellets, Granulate oder Minitabletten, ein transdermales Therapiesystem z. B. in Form eines Pflasters oder eines Ausstrichs, Kapseln, Teile von Kapseln oder sonstigen Arzneiformen, Sachets, Trockensäfte, Suppositorien, Vaginalia oder Implantate handelt.

13. Arzneiform nach Anspruch 11 oder 12, **dadurch gekennzeichnet, daß** ein Wirkstoff aus der Wirkstoffklasse der Abführmittel, Analgetika, Antibiotika, Antirheumatika, Antiallergika, Antiarrhythmika, Antibiotika, Antiepileptica, Betarezeptorenblocker, Calciumkanalblocker, Chemotherapeutika, Enzyme, Extrakte, Hemmstoffe des Renin-Angiotensin-Systems, Broncholytika/Antiasthmatika Cholinergika, Diuretika, Durchblutungsfördernde Mittel, Gichtmittel Grippemittel, Koronarmittel, Osteoporosemittel (Biphosphonate), Lipidsenker, Magen-Darmmittel, Peptide, Proteine, Protonenpumpenblocker, Psychopharmaka" Thrombozytenaggregationshemmer Urologika Venetherapeutika, Vitamine und Mineralien enthalten ist.

14. Arzneiform nach Anspruch 13, **dadurch gekennzeichnet, daß** als Wirkstoff Paroxetin, Reboxetin Morphin und dessen Derivate,Tramadol, Bisacodyl, Natriumfluorid Acamprosat-Ca, Digitoxin, Dimethicon. Kolibakterien, Liponsäure, Methenamin, Budenosid, Acetylsalicylsäure, Diclofenac, Flurbiprophen, Indometacin, Lonazolac, Hydrocortison, Ibuprofen, Ketoprofen,Prdnisolon, Propyphenazon, Naproxen, Paracetamol, Flurbiprofen, Dimetinden, Chinidin, Metoprolol, Propranolol, Oxprenolol, Pindolol, Atenolol, Metoprolol, Disopyramid, Verapamil, Diltiazem, Gallopamil, Nifedipin, Nicardipin, Nisoldipin, Nimodipin, Amlodipin, Theophyllin, Salbutamol, Terbutalin, Ambroxol, Aminophyllin, Carbamazepin, Alendronat, Etidronat, Clodronat, Pamidronat, Ibandronate Cholintheophyllinat, Pyridostigmin, Piretanid, Furosemid, Pentoxifyllin, Naftidrofuryl, Buflomedil, Xantinolnicotinat, Bencyclan, Allopurinol, Norephedrin, Clorphenamin Isosorbidmononitrat, Isosorbiddinitrat, Glyceroltrinitrat, Molsidomin, Bezafibrat, Fenofibrat, Gemfibrozil, Cerivastatin, Pravastatin, Fluvastatin, Lovastatin, Atorvastatin, Simvastatin, 5-Aminosalicylsäure, Sulfasalazin, Budenosid, Natamycin, Preglumetacin Sulfasalacin, Nitrofurantion Xantinol, Metoclopramid, Amitriptylin, Dibenzepin, Venlafaxin, Thioridazin, Oxazepam, Omeprazol, Lanzoprazol, Pantoprazol, Rabeprazol, Perprazol, Esomprazol, , Nitrofurantoin, Rutosid, Knoblauch, Aescin,Bromelain, Pankreatin oder Trypsin, ein Insulin, ein Human Growth Hormon (hGH), Corbaplatin, Intron A, Calcitonin, Cromalyn, ein Interferon, ein Calcitonin, Granulocyte Colony Stimulating factor (G-CSF), ein Interleukin, ein Kinin, Parathyroidhormone, Glucagon, Pindolol, ProSomatostatin, ein Somatostatin, Detirelix, Cetrorelix, Vasopressin, 1-Deaminocysteine-8-D-arginine-Vasopressin, Leuprolidacetat oder ein Antigen, das aus Gräsern oder anderen Pflanzen, wie z. B. Roggen, Weizen, Gerste, Hafer, Bermuda Gras, Zinnkraut, Ahorn, Ulme, Eiche, Platane, Pappel, Zeder, Zinnkraut, Disteln gewonnen wurde, 1gG, spezifische Impfstoffe oder monoclonale Antikörper, pflanzliche Trockenextrakt, Ascorbinsäure, Aspartamsäure, Valproinsäure Zink, und Kalium, Natrium, Lithium und deren pharmazeutisch verwendete Salze.

15. Copolymer, geeignet zur Herstellung einer Arzneiform gemäß den Ansprüchen 11 bis 14.

16. Copolymer nach Anspruch 15, **dadurch gekennzeichnet, daß** es in Form eines teilneutralisierten Pulvers vorliegt.

17. Copolymer nach Anspruch 15 oder 16, **dadurch gekennzeichnet, daß** es in Pulverform in Mischung mit üblichen pharmazeutischen Zuschlagstoffen in einer leicht redispergierbaren Form vorliegt.

18. Verwendung des Copolymeren nach einem oder mehreren der Ansprüche 15 bis 17 in einem Verfahren zur Herstellung einer Arzneiform nach Anspruch 1 bis 10.

19. Verwendung des Copolymeren nach Anspruch 15 bis 17 als Bestandteil oder Inhaltsstoff von Kosmetika oder Nahrungsergänzungsstoffen

## Claims

1. Process for producing a coated drug form or a drug form in the form of an active ingredient matrix, by processing a copolymer, an active pharmaceutical ingredient, a core if present and/or pharmaceutically customary excipients in a conventional manner by melting, injection moulding, extrusion, wet granulation, casting, dipping, spreading, spraying or compression to form a coated drug form and/or to form an active ingredient matrix,
**characterized in that** a copolymer is used which is composed of
20 to 33% by weight methacrylic acid and/or acrylic acid,
5 to 30% by weight methyl acrylate and
20 to 40% by weight ethyl acrylate and, greater than 10 to 30% by weight butyl methacrylate and,
if desired,
0 to 10% by weight further vinylically copolymerizable monomers,
where the proportions of the monomers add up to 100% by weight,
with the proviso that the glass transition temperature of the copolymer is 55 to 70°C.

2. Process according to Claim 1, **characterized in that** 5 to 25% by weight of a plasticizer are added to the coating agent and binder.

3. Process according to Claim 1 or 2, **characterized in that** the copolymer is used in the form of a dispersion for producing the drug form.

4. Process according to Claim 3, **characterized in that** the dispersion has been obtained by redispersing a freeze-dried or spray-dried copolymer powder.

5. Process according to Claim 3 or 4, **characterized in that** from 2 to 10% by weight of the copolymer present in the dispersion is in partly neutralized form.

6. Process according to Claim 5, **characterized in that** the partial neutralization has been brought about by adding NaOH.

7. Process according to one or more of Claims 3 to 6, **characterized in that** an anionic emulsifier is present in an amount of from 0.1 to 2% by weight.

8. Process according to Claim 7, **characterized in that** sodium lauryl sulphate is present as emulsifier.

9. Process according to one or more of Claims 1 to 8, **characterized in that** the copolymer, if desired in combination with pharmaceutically customary additives, is processed to form shaped bodies and an active pharmaceutical ingredient is enclosed therein.

10. Process according to Claim 9, **characterized in that** the shaped body is a capsule, the part of a capsule or a weldable film.

11. Drug form obtainable according to one or more of Claims 1 to 10.

12. Drug form according to Claim 11, **characterized in that** it comprises tablets, including tablets with delayed or accelerated disintegration, minitablets, pellets, tablets compressed from pellets, pellets, granules or minitablets as a filling in capsules, a transdermal therapy system in the form, for example, of a patch or a smear, capsules, parts of capsules or other drug forms, sachets, dried juices, suppositories, pessaries or implants.

13. Drug form according to Claim 11 or 12, **characterized in that** an active ingredient is present from the active ingredients class of the laxatives, analgesics, antibiotics, antirheumatics, antiallergics, antiarrhythmics, antibiotics, antiepileptics, beta-receptor blockers, calcium channel blockers, chemotherapeutics, enzymes, extracts, inhibitors of the renin-angiotensin system, broncholytics/antasthmatics, cholinergics, diuretics, circulation promoters, gout agents, influenza agents, coronary agents, osteoporosis agents (biphosphonates), lipid reducers, gastrointestinal agents, peptides, proteins, proton pump blockers, psychopharmaceuticals, platelet aggregation inhibitors, urological agents, venous therapeutic agents, vitamins and minerals.

14. Drug form according to Claim 13, **characterized in that** it comprises as active ingredient paroxetine, reboxetine, morphine and its derivatives, tramadol, bisacodyl, sodium fluoride, acamprosate Ca, digitoxin, dimethicone, coli bacteria, lipoic acid, methenamine, budenoside, acetylsalicylic acid, diclofenac, flurbiprophen, indometacin, lonazolac, hydrocortisone, ibuprofen, ketoprofen, prednisolone, propyphenazone, naproxen, paracetamol, flurbiprofen, dimetindene, quinidine, metoprolol, propranolol, oxprenolol, pindolol, atenolol, metoprolol, disopyramide, verapamil, diltiazem, gallopamil, nifedipine, nicardipine, nisoldipine, nimodipine, amlodipine, theophylline, salbutamol, terbutaline, ambroxol, aminophylline, carbamazepine, alendronate, etidronate, clodronate, pamidronate, ibandronate, choline theophyllinate, pyridostigmine, piretanide, furosemide, pentoxifylline, naftidrofuryl, buflomedil, xantinol nicotinate, bencyclane, allopurinol, norephedrine, clorphenamine isosorbide mononitrate, isosorbide dinitrate, glycerol trinitrate, molsidomine, bezafibrate, fenofibrate, gemfibrozil, cerivastatin, pravastatin, fluvastatin, lovastatin, atorvastatin, simvastatin, 5-aminosalicylic acid, sulphasalazine, budenoside, natamycin, preglumetacin, sulphasalacine, nitrofurantion, xantinol, metoclopramid, amitriptyline, dibenzepine, venlafaxin, thioridazine, oxazepam, omeprazole, lanzoprazole, pantoprazole, rabeprazole, perprazole, esomprazole, nitrofurantoin, rutoside, garlic, aescin, bromelaine, pancreatin or trypsin, an insulin, a human growth hormone (hGH), corbaplatin, intron A, calcitonin, cromalyn, an interferon, a calcitonin, granulocyte colony stimulating factor (G-CSF), an interleukin, a kinine, parathyroid hormones, glucagon, pindolol, prosomatostatin, a somatostatin, detirelix, cetrorelix, vasopressin, 1-deaminocysteine-8-D-arginine vasopressin, leuprolide acetate or an antigen obtained from grasses or other plants, such as rye, wheat, barley, oats, Bermuda grass, horsetail, maple, elm, oak, plane, poplar, cedar, horsetail, thistles, IgG, specific vaccines or monoclonal antibodies, dry plant extract, ascorbic acid, aspartamic acid, valproic acid zinc, and potassium, sodium, lithium and their salts used pharmaceutically.

15. Copolymer suitable for producing a drug form according to Claims 11 to 14.

16. Copolymer according to Claim 15, **characterized in that** it is in the form of a partially neutralized powder.

17. Copolymer according to Claim 15 or 16, **characterized in that** it is in powder form in a mixture with customary pharmaceutical excipients in a readily redispersible form.

18. Use of the copolymer according to one or more of Claims 15 to 17 in a process for producing a drug form according to Claim 1 to 10.

19. Use of the copolymer according to Claim 15 to 17 as a constituent or ingredient of cosmetics or a nutritional supplement.

## Revendications

1. Procédé de fabrication d'une forme de médicament enrobée ou d'une forme de médicament sous la forme d'une matrice contenant une substance active, dans lequel un copolymère, une substance active pharmaceutique, un noyau présent si nécessaire et/ou les adjuvants pharmaceutiques habituels sont traités d'une manière connue en soi par le biais d'une fusion, d'un moulage par injection, d'une extrusion, d'une granulation à l'état humide, d'une coulée, d'un trempage, d'une enduction, d'une pulvérisation ou d'un pressage pour former une matrice contenant une substance active,
**caractérisé en ce qu'**on utilise un copolymère, lequel est constitué de
20 à 33 % en poids d'acide méthacrylique et/ou d'acide acrylique,
5 à 30 % en poids d'acrylate de méthyle et
20 à 40 % en poids d'acrylate d'éthyle et
plus que 10 à 30 % en poids de méthacrylate de butyle et,
si nécessaire
0 à 10 % en poids d'autres monomères vinyliques copolymérisables,
les proportions de monomères s'additionnant pour donner 100 % en poids,
dans la mesure où la température de vitrification du copolymère s'élève à 55 à 70°C.

2. Procédé selon la revendication 1, **caractérisé en ce que** 5 à 25 % en poids de plastifiant sont ajoutés à l'excipient et à l'agent d'enrobage.

3. Procédé selon la revendication 1 ou 2, **caractérisé en ce que** pour la fabrication d'une forme de médicament, le copolymère est utilisé sous la forme d'une dispersion.

4. Procédé selon la revendication 3, **caractérisé en ce que** la dispersion a été obtenue par la redispersion d'une poudre de copolymère lyophilisée ou séchée par pulvérisation.

5. Procédé selon la revendication 3 ou 4, **caractérisé en ce que** le copolymère contenu dans la dispersion sous forme partiellement neutralisée représente de 2 à 10 % en poids.

6. Procédé selon la revendication 5, **caractérisé en ce que** la neutralisation partielle a été produite en ajoutant du NaOH.

7. Procédé selon l'une quelconque ou plusieurs des revendications 3 à 6, **caractérisé en ce qu'**un émulsifiant anionique, représentant entre 0,1 et 2 % en poids, est compris.

8. Procédé selon la revendication 7, **caractérisé en ce que** du lauryle sulfate de sodium est compris en tant qu'émulsifiant.

9. Procédé selon l'une quelconque ou plusieurs des revendications 1 à 8, **caractérisé en ce que** le copolymère, si nécessaire en combinaison avec des additifs pharmaceutiques habituels, est transformé en corps moulé, et une substance pharmaceutique active y est ajoutée.

10. Procédé selon la revendication 9, **caractérisé en ce que** le corps moulé est une capsule, la partie d'une capsule ou une feuille soudable.

11. Forme de médicament pouvant être fabriquée selon l'une quelconque ou plusieurs des revendications 1 à 10.

12. Forme de médicament selon la revendication 11, **caractérisée en ce qu'**il s'agit de comprimés, de comprimés ayant une décomposition différée ou accélérée, de pastilles, de comprimés injectés à partir de pastilles, de mini-comprimés, de pastilles, granulés ou mini-comprimés remplis dans des capsules, d'un système thérapeutique transdermique par exemple sous forme d'un emplâtre ou d'une application, de capsules, de parties de capsules ou d'autres formes de médicaments, de sachets, de jus secs, de suppositoires, de gaines ou d'implants.

13. Forme de médicament selon la revendication 11 ou 12, **caractérisée en ce qu'**une substance active est incluse, provenant de la catégorie des substances actives des laxatifs, analgésiques, antibiotiques, antirhumatismaux, antiallergiques, antiarythmisants, antibiotiques, anticonvulsivants, bêtabloquants, bloquants de canal à calcium, agents de chimiothérapie, enzymes, substances relargables, inhibiteurs de système de rénine-angiotensine, broncho-dilatateurs/antiasthmatiques cholinergiques, diurétiques, moyens de perfusion, agent de diathèse arthritique, agent grippal, agent coronaire, agent d'ostéoporose (biphosphonate), réducteur de lipide, agent d'estomac-intestin, peptides, protéines, bloquants de pompage des protons, psychotropes, médications urologiques et veineuses d'inhibition d'agrégation de thrombocyte, vitamines et minéraux.

14. Forme de médicament selon la revendication 13, **caractérisée en ce que** sont incluses en tant que substance active : la paroxétine, la réboxétine, la morphine et ses dérivés, le tramadol, le bisacodyl, acamprosate-Ca de fluorure de sodium, la digitoxine, le diméticone, les colibactéries, l'acide lipoïque, la méthénamine, le budénoside, l'acide acétylsalicylique, le diclofénac, le flurbiprofène, l'indométhacine, le lonazolac, l'hydrocortisone, l'ibuprofène, le kétoprofène, le prednisolone, le propyphénazone, le naproxène, le paracétamol, le flurbiprofène, le dimétindène, la quinidine, le métoprolol, le propranolol, l'oxprénolol, le pindolol, l'aténolol, le métoprolol, le disopyramide, le vérapamil, le diltiazème, le gallopamil, la nifédipine, la nicardipine, la nisoldipine, la nimodipine, l'amlodipine, la théophylline, la salbutamol, la terbutaline, l'ambroxol, l'aminophylline, la carbamazépine, l'alendronate, l'étidronate, le clodronate, le pamidronate, l'ibandronate, le cholinthéophyllinate, la pyridostigmine, le pirétanide, le furosémide, la pentoxifylline, le naftidrofuryl, le buflomédil, le nicotinat xantinol, le bencyclane, l'allopurinol, la noréphédrine, mononitrate d'isosorbide de chlorphénamine, l'isosorbidinitrate, le glycérotrinitrate, la molsidomine, le bézafibrate, le fénofibrate, le gemfibrozile, la cérivastatine, la pravastatine, la fluvastatine, la lovastatine, l'atorvastatine, la simvastatine, l'acide aminosalicylique-5, la sulfasalazine, le budénoside, la natamycine, la sulfasalazine de préglumétacine, le xantinol de nitrofurantoïne, le metoclopramide, l'amitriptyline, la dibenzépine, la venlafaxine, le thioridazine, l'oxazépam, l'oméprazole, la lansoprazole, le pantoprazole, le rabéprazole, le perprazole, l'esomprazole, la nitrofurantoïne, le rutoside, l'ail, l'aescine, le bromélaïne, la pancréatine ou trypsine, une insuline, l'hormone somatotrope, la carboplatine, l'intron A, la calcitonine, la cromalyne, un interféron, une calcitonine, le facteur de croissance hématopoïétique G-CSF, une interleukine, une kinine, l'hormone parathyroïde, le glucagon, le pindolot, la prosomatostatine, une somatostatine, le détirelix, le cétrorelix, la vasopressine, la vasopressine-arginine-8-D déaminocystéine-1, l'acétate de leuprolide ou un antigène, des plantes provenant des graminées ou autres comme par exemple le seigle, le froment, l'orge, l'avoine, le cynodon, la prêle des marais, l'érable, l'orme, le chêne, le platane, le peuplier, le cèdre, la prêle des marais transformée en chardon, des vaccins spécifiques ou des anticorps monoclonaux type IgG, un extrait sec végétal, l'acide ascorbique, l'acide aspartique, le zinc d'acide valproïque, et le potassium, le sodium, le lithium et leurs sels utilisés au niveau pharmaceutique.

15. Copolymère, approprié pour la fabrication d'une forme de médicament selon l'une quelconque des revendications 11 à 14.

16. Copolymère selon la revendication 15, **caractérisé en ce qu'**il se présente sous la forme d'une poudre partiellement neutralisée.

17. Copolymère selon la revendication 15 ou 16, **caractérisé en ce qu'**il se présente sous forme de poudre mixée avec des adjuvants pharmaceutiques habituels sous une forme légèrement redispersable.

18. Utilisation de copolymères selon l'une quelconque ou plusieurs des revendications 15 à 17 dans un procédé de fabrication d'une forme de médicament selon la revendication 1 à 10.

19. Utilisation de copolymères selon la revendication 15 à 17 comme élément constitutif ou ingrédient de cosmétiques ou de compléments alimentaires.
